# EUROPEAN PATENT APPLICATION

(11) **EP 4 640 341 A1**
(43) Date of publication of application: **29.10.2025**
(21) Application number: 25170421.9
(22) Date of filing: 14.04.2025
(51) Int. Cl.: B22F 3/02, B22F 3/10, B22F 3/17

(54) **POWDER METAL FORGING HEAT SAMPLING COMPONENT**

(30) Priority: 16.04.2024 US 202418636796
(71) Applicant: RTX Corporation, Farmington, CT 06032 (US)
(72) Inventor: Vinson, Elizabeth F., Broad Brook, 06016 (US); Hjelm, Scott D., Somers, 06071 (US); Goetz, Robert Louis, Dresden, 43821 (US)
(74) Representative: Schmitt-Nilson Schraud Waibel Wohlfrom Patentanwälte Partnerschaft mbB

(57) **Abstract**

A heat sampling component for powder metal forging including a heat sampling component formed from a portion of a heat of powder forging; at least one inclusion formed within the heat sampling component; and a part location defined within the heat sampling component, wherein the part location includes a part.

## Description

The present disclosure is directed to an improved process and a generic forged shape configured to sample material for inclusions that could become process anomalies during forging and heat treatment.

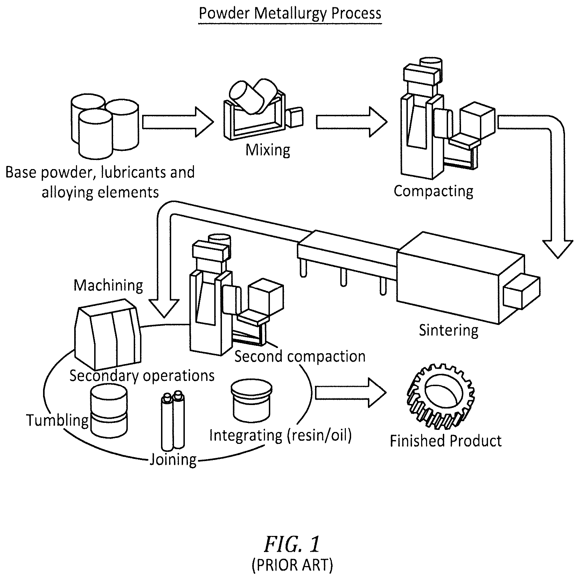

Referring to Fig. 1, a powder metallurgy process is shown. During powder metallurgy source materials in powder form are selected. The source materials can include base materials, lubricants, and alloying elements. The powder materials are mixed and then compacted. Typically, the materials are compacted in a die through the application of high pressure. The compacted materials are sintered to form a part. Sintering includes heating the compacted materials in a controlled atmosphere. During sintering, the surfaces of the particles are bonded and desirable properties are achieved. After sintering, the part can be machined as needed. The machined part can be used as a finished product.

The powder metallurgy process can be conducted in large batches and production runs. A campaign can be a production of from about one million pounds to about one half million pounds of material. Heats of powder or simply a heat can be on the order of thousands of pounds of material being powder metal forged. A forging can be on the order of about one hundred pounds of powder metallurgy materials being forged. It is possible during these large batches to have contamination enter the powder metal materials. The contamination can be elements, such as impurities, that can cause defects in the final forging material. The contamination can be found from original sources or even come from production equipment contamination. The contaminants can be passed along through the process and end up being formed within a portion of an individual part. The contaminant can be an inclusion which can adversely impact the part by creating an imperfection in the material structure. The imperfection can result in cracking, voiding, and other material structure defects. Heats of powder generally produce similar parts - same forgings per heat of material. Some components may have critical defects that are less detectable than other components. For example, cover plate embedded defect inspections are more challenged than larger rotor forging inspections.

Detecting the inclusions becomes an important step in the process, particularly detecting the inclusions prior to machining steps or even as late as when parts are being released to a warehouse.

In accordance with the present disclosure, there is provided a heat sampling component for powder metal forging comprising a heat sampling component formed from a portion of a heat of powder forging; at least one inclusion formed within the heat sampling component; and a part location defined within the heat sampling component, wherein the part location includes a part.

Particular embodiments further may include at least one, or a plurality of, the following optional features, alone or in combination with each other:

A further embodiment of any of the foregoing embodiments may additionally and/or alternatively include the heat sampling component having a predetermined shape configured to predict the location of possible anomalies in the heat of powder forging material.

A further embodiment of any of the foregoing embodiments may additionally and/or alternatively include the predetermined shape being configured responsive to the shape of the part.

A further embodiment of any of the foregoing embodiments may additionally and/or alternatively include the predetermined shape being configured to enhance a predictive analysis tool function.

A further embodiment of any of the foregoing embodiments may additionally and/or alternatively include the heat sampling component for powder metal forging further comprising a predictive analysis tool in operative communication with the heat sampling component.

A further embodiment of any of the foregoing embodiments may additionally and/or alternatively include the predictive analysis tool being configured to analyze the heat sampling component and provide predictive inclusion locations.

A further embodiment of any of the foregoing embodiments may additionally and/or alternatively include that the predictive inclusion locations indicate a location of potential inclusions within the heat sampling component.

In accordance with the present disclosure, there is provided a heat sampling component system for powder metal forging comprising a heat sampling component formed from a portion of a heat of powder forging; a predictive analysis tool in operative communication with the heat sampling component; at least one inclusion formed within the heat sampling component; and a part location defined within the heat sampling component, wherein the part location includes a part.

Particular embodiments further may include at least one, or a plurality of, the following optional features, alone or in combination with each other:

A further embodiment of any of the foregoing embodiments may additionally and/or alternatively include the heat sampling component having a predetermined shape configured to predict the location of possible anomalies in the heat of powder forging material.

A further embodiment of any of the foregoing embodiments may additionally and/or alternatively include the predictive analysis tool being configured to analyze the heat sampling component and provide predictive inclusion locations.

A further embodiment of any of the foregoing embodiments may additionally and/or alternatively include that the predictive inclusion locations indicate a location of potential inclusions within the heat sampling component.

A further embodiment of any of the foregoing embodiments may additionally and/or alternatively include the heat sampling component having a predetermined shape configured to predict the location of possible anomalies in the heat of powder forging material.

A further embodiment of any of the foregoing embodiments may additionally and/or alternatively include the predetermined shape being configured to enhance the predictive analysis tool function by more closely resembling the shape of the part.

In accordance with the present disclosure, there is provided a process for a heat sampling component system for powder metal forging comprising identifying a heat of powder forging; forming a heat sampling component for the heat of powder forging; locating a part having a part location within the heat sampling component; generating a predictive inclusion location associated with the heat sampling component; and comparing the part location with the predictive inclusion location.

Particular embodiments further may include at least one, or a plurality of, the following optional features, alone or in combination with each other:

A further embodiment of any of the foregoing embodiments may additionally and/or alternatively include the process further comprising coupling a predictive analysis tool in operative communication with the heat sampling component.

A further embodiment of any of the foregoing embodiments may additionally and/or alternatively include the predictive analysis tool being configured to generate the predictive inclusion location.

A further embodiment of any of the foregoing embodiments may additionally and/or alternatively include the process further comprising forming a predetermined shape from the heat sampling component, the predetermined shape configured to predict the location of possible anomalies in the heat of powder forging material.

A further embodiment of any of the foregoing embodiments may additionally and/or alternatively include the process further comprising configuring the predetermined shape to enhance the predictive analysis tool function to generate the anomalies within the forging.

A further embodiment of any of the foregoing embodiments may additionally and/or alternatively include the process further comprising mitigating a chance of forming a part containing inclusions.

A further embodiment of any of the foregoing embodiments may additionally and/or alternatively include the step of mitigating comprises recycling the heat of powder forging in the absence of machining parts from the heat of powder forging.

In order to ensure robust monitoring of heats of material, a generic rotor bore like component could be included in each heat to keep from mixing part numbers in heats, but also ensuring every heat has a sample of material that challenges the anomalies of interest and is inspectable to the desired capability.

Other details of the improved process and generic forged shape configured to sample material for inclusions are set forth in the following detailed description and the accompanying drawings wherein like reference numerals depict like elements.
Fig. 1 is a prior art schematic representation of a powder metallurgy process.
Fig. 2 is a schematic representation of an exemplary heat of powder forging.
Fig. 3 is a schematic representation of an exemplary embodiment of a forged hollow cylinder.
Fig. 4 is a schematic representation of a cross-section of the forged hollow cylinder of Fig. 3.
Fig. 5 is a schematic representation of an exemplary forged sample shape of the heat sampling component.
Fig. 6 is a schematic representation of an exemplary forged sample shape of the heat sampling component.
Fig. 7 is a schematic representation of an exemplary forged sample shape of the heat sampling component.
Fig. 8 is a process map.

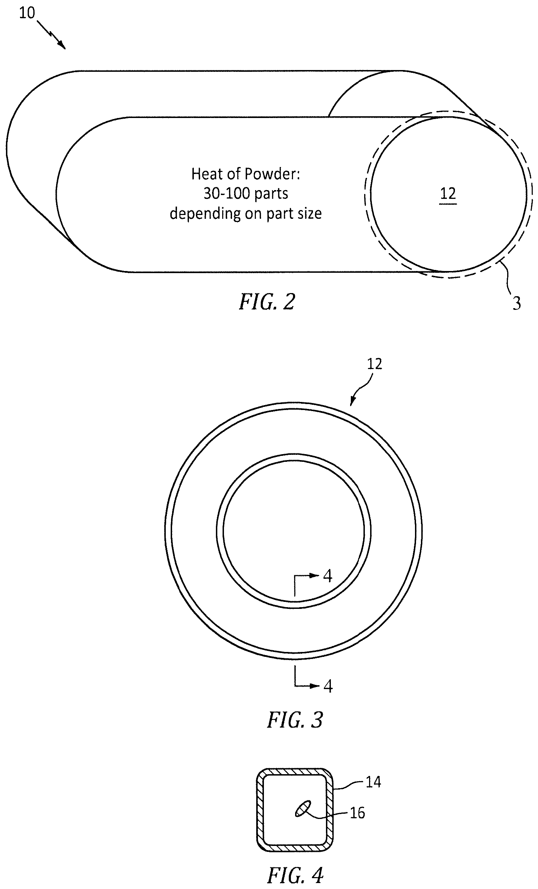

Referring now to Fig. 2, there is illustrated an exemplary heat of powder forging 10. Exemplary powders that can be used to form powder forging 10 can include, but are not limited to, Ni-based superalloys (with or without additional alloying elements). The heat of powder forging 10 can be processed to create on the order of 30 to 100 individual parts depending on the part size. A portion 12 of the heat of powder forging 10 can be removed as seen in Fig. 3. The portion 12 is shown as a right circular hollow cylinder shape, however many different shapes can be utilized. Fig. 4 shows a heat sampling component 14 as a cross-section cut from the portion 12. The heat sampling component 14 can be analyzed through various forms of scanning and detection to indicate the presence of a reactive inclusion 16. The reactive inclusion 16 can be a precursor to process anomalies, such as cracking, voiding, and the like, during the forging process and heat treat process. Reactive inclusion(s) 16 can include for example non-metallic inclusions (NMIs). For example, and while not to be construed as limiting, Al₂MgO₄, and/or aluminum oxides (such as Al₂O₃) can become reactive and have reduced properties under certain conditions or circumstances (e.g., including when phosphorus and/or silicon are near such inclusions) such that they can be susceptible to cracking.

The capacity to detect and/or predict the presence of the inclusion 16 can provide advantages in the powder metal forging process.

The heat sampling component 14 can be shaped in a variety of designs depending on the shape of the intended part to be formed from the heat of powder forging 10. Each heat of powder forging 10 shape per part creates challenges to the materials. Each shape per part challenges the material differently during the forging process. The heat sampling component 14 shape is created to predict the location of possible anomalies in the material to better understand the heat of powder forging 10 as early as possible in the forging process. The earlier the inclusion 16 can be detected/predicted in the forging process the greater the cost savings and reduction in future defects. The heat sampling component 14 shape can be created in order to target a particular parameter to activate the critical defects. In other words, the forging/heat treat process can produce anomalies that are difficult to detect at the heat of powder forging 10 scale. But by properly sizing the heat sampling component 14, the anomalies small critical size would be more easily detectable within the heat sampling component 14.

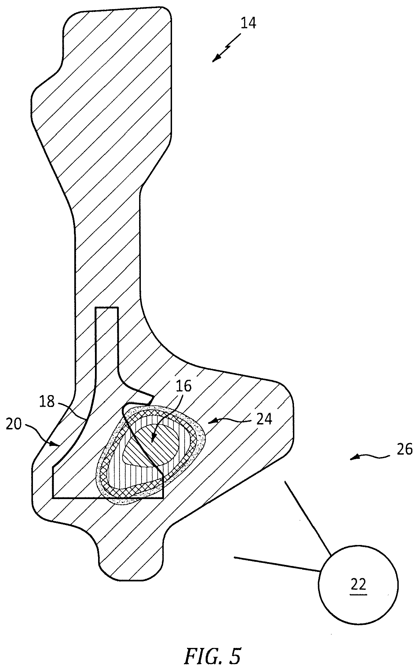

Referring also to Fig. 5 to Fig. 7., exemplary shapes of the heat sampling component 14 are shown. For example, the Fig. 5 illustration shows a heat sampling component 14 with a particular predetermined shape. The heat sampling component 14 can be used to indicate potential locations of the inclusion 16. A part 18 cross-section shape is shown within the heat sampling component 14. The part 18 shape is shown within the heat sampling component 14 at a part location 20.

A predictive analysis tool 22 can be employed to analyze the heat sampling component 14 and provide predictive inclusion locations 24. The predictive inclusion locations 24 can show the location of potential anomalies inclusions 16 within the heat sampling component 14. The addition of the predictive analysis tool 22 with the heat sampling component 14 can create a heat sampling component system for powder metal forging 26. Using material behavior data and manufacturing process parameters, the predictive analysis tool 22 can estimate the size for different portions of the sampling component 14. The size can be the anomaly that could become life limiting for the part 18. By evaluating the entire manufacturing process the smallest critical anomaly can be predicted. Material creep crack growth threshold and strength can be used to evaluate the component stresses throughout the manufacturing process in order to predict the minimum defect for each region of the part 18. The parameters of the prediction can be the material and the manufacturing process. The manufacturing process includes the changing of the shape from the billet to the heat sampling component 14. The predictive analysis tool 22 can be used to refine the geometry and the process to maximize the region of the heat sampling component that challenges the typical anomalies.

The predictive inclusion locations 24 can be of varying size and number and reflect a relative likelihood of potential inclusions 16. The darker shade shown at Fig. 5 within the predictive inclusion locations 24 can be representative of a higher likelihood of an inclusion 16 and the lighter shaded regions can be representative of less likely locations of an inclusion 16.

As seen in Fig. 5, the part 18 has a portion that is overshadowed by some of the predictive inclusion locations 24. Thus, operators can be informed of the potential of an inclusion 16 being located within the part 18 during the forging/heat treatment process. Mitigation steps can be initiated to prevent the inclusion 16 for a particular heat of powder forging 10.

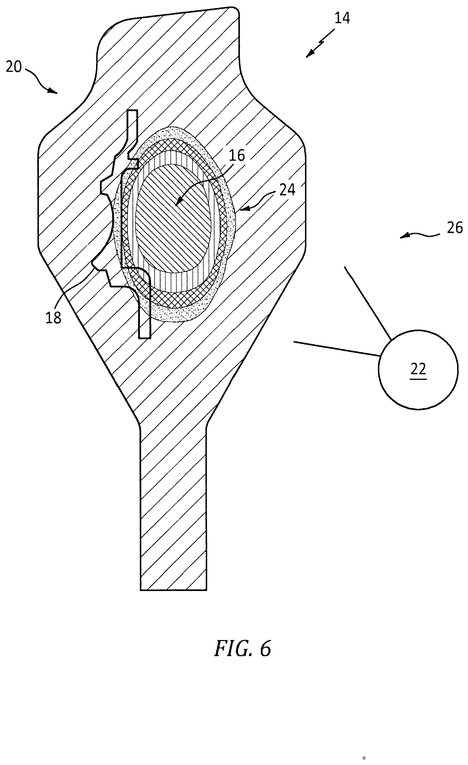

Also as seen in Fig. 6, the heat sampling component 14 is shown with a part 18 within the boundary of the heat sampling component 14. The predictive analysis tool 22 can be employed to analyze the heat sampling component 14 and provide the predictive inclusion locations 24. The predictive inclusion locations 24 can show the location of potential inclusions 16 within the heat sampling component 14. The part 18 of Fig. 6 is shown overshadowed by only a portion of the predictive inclusion location 24. Since the predictive inclusion location 24 does not overlap the part 18 to a large degree, it is possible that the operators can proceed further with the forging and heat treatment process based on the predictions.

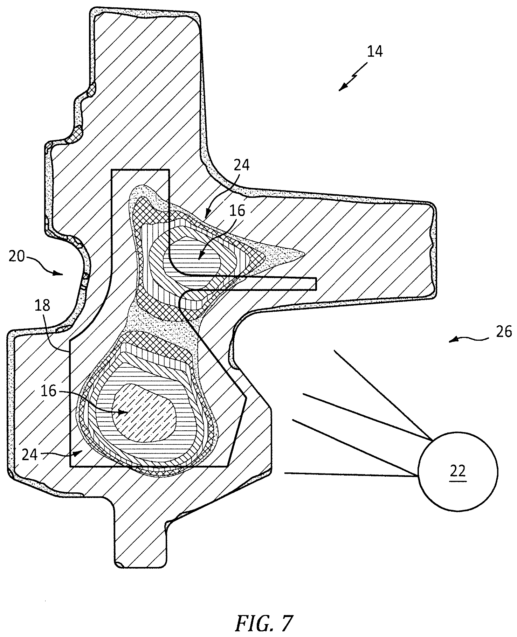

Also as seen in Fig. 7, the heat sampling component 14 is shown with a part 18 within the boundary of the heat sampling component 14. The predictive analysis tool 22 can be employed to analyze the heat sampling component 14 and provide the predictive inclusion locations 24. The predictive inclusion locations 24 can show the location of potential inclusions 16 within the heat sampling component 14. The part 18 of Fig. 7 is shown overshadowed by two of the predictive inclusion locations 24. The operators may take action to recycle the heat of powder forging 10, since there are predictive inclusion locations 24 of such quantities and locations overshadowing the part 18.

In each of the examples shown in Fig. 5 to Fig. 7 the heat sampling component 14 is shaped to optimize the capacity of the predictive analysis tool 22 to produce the predictive inclusion locations 24 relative to the part location 20. It is contemplated that a relevant volume of the heat of powder forging 10 can be challenged by employing the appropriately shaped heat sampling component 14 to screen for the potential inclusions 16. The heat sampling component 14 can be designed to cover any heat of powder forging 10 sizes. In an exemplary embodiment, the heat sampling component 14 can be sized with a generic geometry configured to detect/predict anomalies of interest in the size ranging from about 0.008 inch to about 0.016 inch.

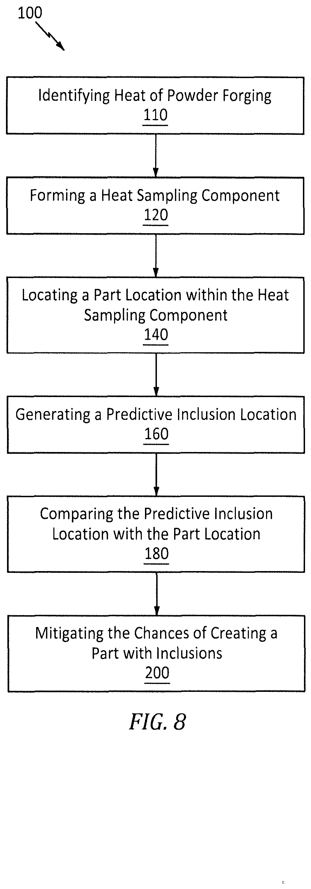

Fig. 8 illustrates a process map for employing the heat sampling component 14. The process 100 includes the step 110 of identifying a heat of powder forging 10. The step 120 includes forming a heat sampling component 14. The heat sampling component 14 can be shaped to detect/predict anomalies and inclusions 16. The step 140 includes locating a part location 20 within the heat sampling component 14. The part location 20 can be indicative of a part 18 that is planned to be formed from the heat of powder forging 10. The next step 160 includes generating a predictive inclusion location 24 by employing the predictive analysis tool 22. The next step 180 is comparing the predictive inclusion location 24 with the part location 20. The next step 200 is mitigating the chances of creating a part with inclusions 16. For example, the heat of powder forging 10 may be recycled back into the process for re-forging to avoid the possibility of having formed a part 18 with an inclusion 16 of consequence.

A technical advantage of the disclosed heat sampling component for powder metal forging includes a more robust comparison across the heat of powder forging.

Another technical advantage of the disclosed heat sampling component for powder metal forging includes a repeatable comparison across the heat of powder forging.

Another technical advantage of the disclosed heat sampling component for powder metal forging includes a heat sampling component with a generic geometry for a given heat of powder forging.

There has been provided a heat sampling component for powder metal forging. While the heat sampling component for powder metal forging has been described in the context of specific embodiments thereof, other unforeseen alternatives, modifications, and variations may become apparent to those skilled in the art having read the foregoing description. Accordingly, it is intended to embrace those alternatives, modifications, and variations which fall within the broad scope of the appended claims.

## Claims

1. A heat sampling component for powder metal forging comprising:
a heat sampling component formed from a portion of a heat of powder forging;
at least one inclusion formed within the heat sampling component; and
a part location defined within the heat sampling component, wherein the part location includes a part.

2. The heat sampling component for powder metal forging according to claim 1, wherein the heat sampling component includes a predetermined shape configured to predict the location of possible anomalies in the heat of powder forging material.

3. The heat sampling component for powder metal forging according to claim 2, wherein the predetermined shape is configured responsive to the shape of the part.

4. The heat sampling component for powder metal forging according to claim 2 or 3, wherein the predetermined shape is configured to enhance a predictive analysis tool function.

5. The heat sampling component for powder metal forging according to any of claims 1 to 4, further comprising:
a predictive analysis tool in operative communication with the heat sampling component.

6. The heat sampling component for powder metal forging according to claim 5, wherein the predictive analysis tool is configured to analyze the heat sampling component and provide predictive inclusion locations.

7. The heat sampling component for powder metal forging according to claim 6, wherein the predictive inclusion locations indicate a location of potential inclusions within the heat sampling component.

8. A heat sampling component system for powder metal forging comprising:
the heat sampling component for powder metal forging according to any of claims 1 to 7, and
a predictive analysis tool in operative communication with the heat sampling component.

9. A process for a heat sampling component system for powder metal forging comprising:
identifying a heat of powder forging;
forming a heat sampling component for the heat of powder forging;
locating a part having a part location within the heat sampling component;
generating a predictive inclusion location associated with the heat sampling component; and
comparing the part location with the predictive inclusion location.

10. The process of claim 9, further comprising:
coupling a predictive analysis tool in operative communication with the heat sampling component.

11. The process of claim 10, wherein the predictive analysis tool is configured to generate the predictive inclusion location.

12. The process of any of claims 9 to 11, further comprising:
forming a predetermined shape from the heat sampling component, the predetermined shape configured to predict the location of possible anomalies in the heat of powder forging material.

13. The process of claim 12, further comprising:
configuring the predetermined shape to enhance the predictive analysis tool function to generate the anomalies within the forging.

14. The process of any of claims 9 to 13, further comprising:
mitigating a chance of forming a part containing inclusions.

15. The process of claim 14, wherein the step of mitigating comprises recycling the heat of powder forging in the absence of machining parts from the heat of powder forging.
